(19) Europäisches Patentamt
European Patent Office
Office européen des brevets

(11) **EP 4 579 672 A1**

(12) **EUROPEAN PATENT APPLICATION**

(43) Date of publication:
**02.07.2025 Bulletin 2025/27**

(51) International Patent Classification (IPC):
**G16C 20/30** (2019.01)   **G16C 20/70** (2019.01)

(21) Application number: **23220577.3**

(52) Cooperative Patent Classification (CPC):
**G16C 20/30;** G06N 3/045; G16C 20/70

(22) Date of filing: **28.12.2023**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Airbus (S.A.S.)**
**31700 Blagnac (FR)**

(72) Inventors:
• **TANDON, Akshat**
  **82024 Taufkirchen (DE)**
• **CARRÉ, Camille**
  **82024 Taufkirchen (DE)**

(74) Representative: **LKGLOBAL**
**Lorenz & Kopf Patentanwalt**
**Attorney at Law PartG mbB**
**Brienner Straße 11**
**80333 München (DE)**

(54) **ASSESSMENT METHOD, PROGRAMM, COMPUTER-READABLE DATA-CARRIER, COMPUTING DEVICE, AND ARRANGEMENT FOR A COMPUTER ASSISTED SUBSTANCE ASSESSMENT**

(57)    An assessment method, a corresponding assessment program (3), a computer-readable data-carrier (4, 4a, 4b) having stored thereon the assessment program (3), a computing device (2) configured to carry out the assessment program (3) and/or comprising the computer-readable data-carrier (4, 4a, 4b), and an assessment arrangement (1) for a computer assisted assessment of at least one property parameter (P) are provided, comprising the steps of obtaining a molecular structure (M) of the chemical substance (X); obtaining at least one reference structure (Q) of at least one reference substance (S) having at least one reference parameter (R), comparing the molecular structure (M) to the at least one reference structure (Q); and deriving the at least one property parameter (P) from the comparison based on the at least one reference parameter (R).

**Fig. 1**

Processed by Luminess, 75001 PARIS (FR)

## Description

Technical Field

The present disclosure relates to the field of computational chemistry in substance development and assessment of their chemical and/or physical properties. In particular, the disclosure relates to an assessment method for computer assisted assessment of at least one property parameter of a chemical substance, to an assessment program, a computer-readable data-carrier, a computing device, and to an assessment arrangement for a computer assisted assessment of at least one property parameter of a chemical substance.

Technical Background

Methods for computer assisted assessment of molecule and material properties are known from the prior art. Such methods allow for predicting respective properties on a theoretical level. The prediction can help in at least a first assessment of a certain chemical and/or physical behaviour of a substance. Based on the prediction, candidates of substances for comprehensive chemical and/or physical assessment through respective laboratory and/or field tests may be shortlisted, thus enabling to streamline respective assessment and testing procedures which can be extensively time and resource consuming.

Wang, Y., Wang, J., Cao, Z. et al., "Molecular contrastive learning of representations via graph neural networks", Nat Mach Intell 4, 279-287 (2022). https://doi.org/10.1038/s42256-022-00447-x, for example, present MolCLR (Molecular Contrastive Learning of Representations via Graph Neural Networks), a self-supervised learning framework that leverages large unlabelled data (~10 million unique molecules). In a MolCLR pre-training, molecule graphs are built, and graph-neural-network encoders are developed to learn differentiable representations. Three molecule graph augmentations are proposed: atom masking, bond deletion and subgraph removal. A contrastive estimator maximizes the agreement of augmentations from the same molecule while minimizing the agreement of different molecules. Experiments are supposed to show that such contrastive learning framework significantly improves the performance of graph-neural-network encoders on various molecular property benchmarks including both classification and regression tasks. MolCLR learns to embed molecules into representations that can distinguish chemically reasonable molecular similarities.

Wang Y, Magar R, Liang C, Barati Farimani A, "Improving Molecular Contrastive Learning via Faulty Negative Mitigation and Decomposed Fragment Contrast", J Chem Inf Model. 2022 Jun 13;62(11):2713-2725. doi: 10.1021/acs.jcim.2c00495. Epub 2022 May 31. PMID: 35638560, state that so-called deep learning has been a prevalence in computational chemistry and widely implemented in molecular property predictions. Recently, self-supervised learning (SSL), especially contrastive learning (CL), has gathered growing attention for the potential to learn molecular representations that generalize to the gigantic chemical space. Unlike supervised learning, SSL can directly leverage large unlabelled data, which greatly reduces the effort to acquire molecular property labels through costly and time-consuming simulations or experiments. However, most molecular SSL methods borrow the insights from the machine learning community but neglect the unique cheminformatics (e.g., molecular fingerprints) and multilevel graphical structures (e.g., functional groups) of molecules. They propose iMolCLR as an improvement of Molecular Contrastive Learning of Representations with graph neural networks (GNNs) in two aspects: (1) mitigating faulty negative contrastive instances via considering cheminformatics similarities between molecule pairs and (2) fragment-level contrasting between intramolecular and intermolecular substructures decomposed from molecules.

Assessment methods, as known from the prior art, do not seem to satisfy all requirements for similarity and/or property predictions for chemical substances. Known methods commonly rely on augmentations. The implemented way of SSL may not achieve desired results in similarity and/or prediction accuracy.

Summary

It may thus be seen as an object to provide alternative methods for similarity and/or property predictions for the assessment of chemical substances. In particular, it may be seen as an object to provide a way of implementing SSL for achieving a relatively high similarity and/or property prediction accuracy while maintaining computational and analytical efficiency. These objects are at least partly achieved by the subject-matter of the independent claims.

According to an aspect, an assessment method for computer assisted assessment of at least one property parameter of a chemical substance is provided, comprising the steps of obtaining a molecular structure of the chemical substance; obtaining at least one reference structure of at least one reference substance having at least one reference parameter, comparing the molecular structure to the at least one reference structure; and deriving the at least one property parameter from the comparison based on the at least one reference parameter.

An assessment program comprising instructions is provided which, when the program is executed by a computing device, cause the computing device to carry out a corresponding assessment method.

A computer-readable data-carrier is provided having stored thereon a corresponding assessment program.

A computing device is provided which is configured to carry out a corresponding assessment program and/or comprises a corresponding computer-readable data-carrier.

An assessment arrangement for a computer assisted assessment of at least one property parameter of a chemical substance is provided, configured to carry out a corresponding assessment method, comprising a corresponding assessment program, comprising a corresponding computer-readable data-carrier, and/or comprising a corresponding computing device.

Alternatively, or additionally, a training method, as well as corresponding training program may be provided, and maybe stored on a computer-readable data-carrier. A computing device may be configured to carry out the training program and/or may comprise respective computer-readable data-carrier. The assessment arrangement thus may comprise a training arrangement, and/or the training arrangement may be provided in addition to the assessment arrangement and may be configured to carry out the training method, may comprise the training program, a corresponding computer-readable data-carrier, and/or the corresponding computing device. The training method may comprise any of the steps of the assessment method as described herein, in particular any steps performed in connection with the prediction algorithm, training parameters, asset or training phases.

The assessment method itself does neither rely on augmentation to find similar molecules, nor on calculating a loss for a pair of structures to be compared to each other. The chemical substances may comprise, for example, halon and other extinguishing agents, PFAS, Bisphenols and other REACH-listed components from resin systems, corrosion inhibitors e.g., chromates, etc., as well as derivates and products of decomposition thereof. The at least one property parameter may represent possible and/or probable toxicity, density, Young's modulus, Tensile strength, boiling points, viscosity, flammability, fuel ignition, corrosivity, optical behaviour, and/or magneticity of the chemical substance.

The proposed solution has the advantage over the prior art, that for each comparing step, a direct comparison between the molecular structure and the reference structure can be carried out. Consequently, a triangular relationship and/or similarity analysis between a molecule structure, and our augmented version thereof, and a reference structure of a reference substance to be compared therewith is not necessary. Any augmentation step of (randomly) altering molecules and respective computational and analytic efforts can be omitted. Thus, a high similarity and/or property prediction accuracy can be achieved with high computational and analytical efficiency.

Further developments can be derived from the dependent claims and from the following description. Features described with reference to devices and arrangements may be implemented as method steps, or vice versa. Therefore, the description provided in the context of the computing device and/or assessment arrangement applies in an analogous manner also to respective methods. In particular, the functions of the computing device and/or of the assessment arrangement and of their or its, respectively, components may be implemented as method steps of the methods and the method steps may be implemented as functions of the computing device and/or of the assessment arrangement.

According to a possible embodiment of an assessment method, the step of comparing involves identifying a similarity between the molecular structure and the at least one reference structure. The similarity can be identified in the respective similarity value can be assigned thereto for the entire molecular structure and reference structure and/or parts thereof. In other words, fingerprints of the molecular structure and the reference structure can be compared to each other in order to identify their similarities or certain similarities of sections thereof. This may further help in providing high similarity and/or property prediction accuracy with high computational and analytical efficiency.

According to a possible embodiment of an assessment method, the step of comparing involves calculating a similarity score of the molecular structure and the at least one reference structure. Any similarity score in general can be applied, in particular a similarity score according to Tanimoto / Jaccard. The similarity score may be based on the similarity value and again may be applied to the entire molecular structure and reference structure and/or parts thereof. This may further help in providing high similarity and/or property prediction accuracy with high computational and analytical efficiency.

According to a possible embodiment of an assessment method, the steps of obtaining the molecular structure and/or of the at least one reference structure involves providing a latent representation of the chemical substance and/or of the at least one reference substance, respectively. The latent representation can be created, generated and/or derived by means of a substance analyser. The latent representation may comprise nodes representing atoms of the molecular structure and/or the at least one reference structure, as well as edges representing bonds between the atoms. The bonds can be interpreted as chemical connections between atoms of a molecule. Thereby, an efficient and reliable way of assessing new, i.e., non-catalogued or unlabelled, chemical substances may be provided. This can help in providing high similarity and/or property prediction accuracy with a desired degree of flexibility and adaptability, while maintaining a preferably high degree of computational and analytical efficiency.

According to a possible embodiment of an assessment method, the step of comparing involves comparing the latent representation of the molecular structure to the latent representation of the at least one reference structure. Certain property parameters to be identified and/or evaluated may be assigned to the latent representations which can be regarded as models of the structures. A desired degree of abstraction and/or generalisation can be applied to the models. This can further help in providing high similarity and/or property prediction accuracy with a desired degree of flexibility and adaptability, while maintaining a preferably high degree of computational and analytical efficiency.

According to a possible embodiment of an assessment method, assessment method further comprises the step of selecting the at least one reference structure of the at least one reference substance from a reference database. The reference database may also hold reference structures of reference substances based on molecule structures which have been analysed and assessed by means of the assessment method. The database thus helps in facilitating SSL.

According to a possible embodiment of an assessment method, at least one the reference structure and/or the at least one reference substance is selected from a reference cluster containing reference structures and/or reference substances, respectively, being relatively similar to each other. The reference database can further contain classifications of reference structures and/or reference substances based on respective reference properties. The clustering and/or the classifications may help in facilitating any selection processes necessary for identifying reference structures, reference substances, and/or reference parameters. This may further help in providing high similarity and/or property prediction accuracy with a high degree of computational and analytical efficiency.

According to a possible embodiment of an assessment method, the step of comparing and/or the step of deriving involves predicting the at least one property parameter by means of a trained prediction algorithm comprising at least one trainable parameter. The trainable parameter may be continuously and/or intermittently trained and adjusted to meet a desired level of similarity and/or property prediction accuracy. Thereby, computational, and analytical efficiency may be further improved.

According to a possible embodiment of an assessment method, the assessment method further comprises the step of training the prediction algorithm by adjusting the trainable parameter through an assessment evaluation of a quality of the prediction of a similarity parameter and/or the at least one property parameter based on a comparison of the at least one reference substance with another reference substance having at least one predetermined similarity score and/or reference parameter, respectively, that serves as a target parameter for the predicted similarity parameter and/or property parameter, respectively. The other words, a predefined similarity parameter and/or at least one property parameter may be used as a target parameter in order to evaluate the quality, i.e., accuracy, of the predicted similarity parameter and/or property parameter, respectively. This helps in further facilitating SSL.

According to a possible embodiment of an assessment method, during a training phase, in particular, a first training phase, the step of comparing the molecular structure to the at least one reference structure is being trained. The first training phase aims at training the assessment of similarities between molecular structures. The training can be based on LSSL, in that a loss function involving a loss L is used in SSL. The loss should be low for all molecular that appears to be compared to each other which is supposed to be having a low similarity value or score and thus may be regarded as rather different to each other. Thereby, in particular a similarity assessment may be trained in a targeted and highly efficient manner.

According to a possible embodiment of an assessment method, during a further training phase, in particular a second training phase, deriving the at least one property parameter from the comparison based on the at least one reference parameter is being trained. The second training phase may follow the first training phase and can be interpreted as a learning phase aiming at assigning properties to the trained assessment of similarities which has been acquired in the first training phase. An N-value score obtained as an output from the first training phase can be used as an input for the second training phase. The N-value score can be a point in a vector space of similarities. Thereby, in particular a property parameter assessment may be trained in a targeted and highly efficient manner.

Consequently, the assessment evaluation may be performed in two steps: A first phase (training phase) may involve using an SSL-loss (LSSL) with similarities for predictions for molecule similarities. A second phase (learning phase) may involve using a loss function, such as a cross entropy loss, indicating a relatively high loss value if a predicted property parameter is relatively different to a target parameter, and indicating a relatively low loss value if a predicted property parameter is relatively similar to a target parameter. Thereby, for example, a semi-supervised learning SSL with a respective loss function LSSL may be applied. A corresponding artificial intelligence (AI) / machine learning (ML) algorithm for comparing the molecular structure to the at least one reference structure and/or for deriving the at least one property parameter from the comparison based on the at least one reference parameter may be implemented.

Brief Description of the Drawings

The subject matter will hereinafter be described in conjunction with the following drawing figures, wherein like numerals denote like elements, and wherein:

Fig. 1 is a schematic presentation of an assessment arrangement for a computer assisted assessment of at least one property parameter of a chemical substance.

Fig. 2 is a schematic illustration of a molecular structure of a chemical substance in chemical nomenclature.

Fig. 3 is a schematic illustration of the molecular structure of the chemical substance X shown in Fig. 2 as a latent representation.

Fig. 4 is a schematic diagram illustrating supervised learning step.

Fig. 5 is a schematic diagram illustrating a downstream learning step.

Fig. 6 is a schematic illustration of a number of molecular structures arranged in a virtual three-dimensional space according to their respective similarities.

Fig. 7 is a schematic block diagram illustrating steps of a first training phase.

Fig. 8 is a schematic block diagram illustrating steps of a second training phase.

Fig. 9 is a schematic block diagram of an application phase.

Fig. 10 is a schematic illustration of the chemical substance X shown in Fig. 2 as another latent representation.

Fig. 11 is a schematic illustration of a different chemical substance as a latent representation

Fig. 12 is a schematic illustration of a different chemical substance as a latent representation which has been derived from the chemical substance X shown in Fig. 10 by augmentation.

Detailed Description of the Drawings

The following detailed description is merely exemplary in nature and is not intended to limit the invention and uses of the invention. Furthermore, there is no intention to be bound by any theory presented in the preceding background or the following detailed description. The representations and illustrations in the drawings are schematic and not to scale. Like numerals denote like elements. A greater understanding of the described subject matter may be obtained through a review of the illustrations together with a review of the detailed description that follows.

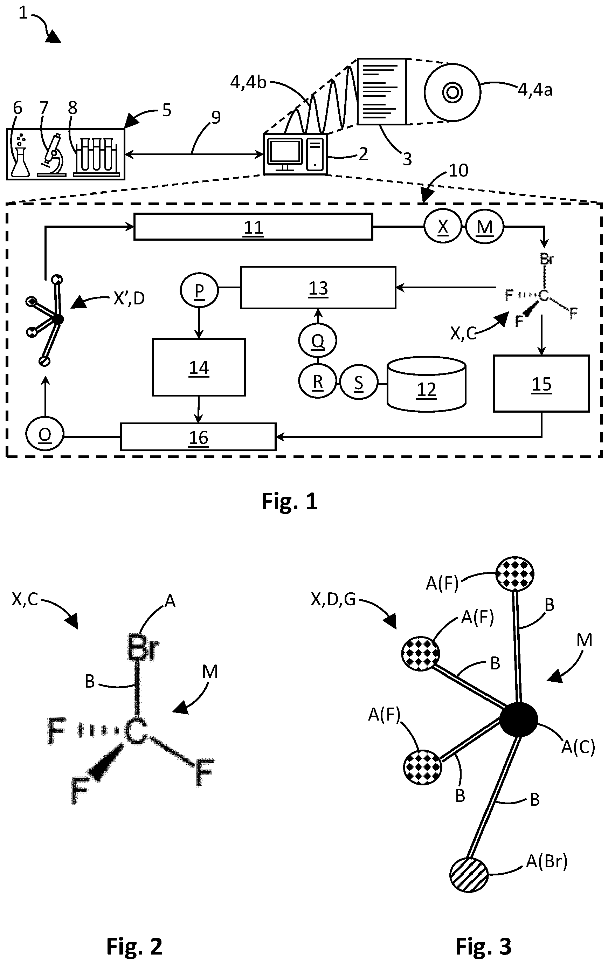

Fig. 1 shows a schematic representation of an assessment arrangement 1 for a computer assisted assessment of at least one property parameter P of a chemical substance X. The assessment arrangement 1 comprises a computing device 2 for implementing an assessment method and/or training method by means of a respective assessment program 3 and/or computer-readable data carrier 4. The computing device 2 is configured to execute the assessment program 3. The computer-readable data carrier 4 has stored thereon the computer assessment program 3 and may take the form of a computer-readable medium 4a and/or data carrier signal 4b.

Furthermore, the assessment arrangement 1 may comprise a laboratory system 5 which may include a reactor device 6, a physical analysis device 7, and/or a chemical analysis device 8. The reactor device 6 may be provided in the form of any kind of chemical reactor for synthesising and/or decomposing the chemical substance X. The physical analysis device 7 may provide any means for a physical analysis of the chemical substance X and thereby obtaining a respective physical property parameter $P_P$ as a form of the property parameter P. The chemical analysis device 8 may provide any means for a chemical analysis of the chemical substance X and thereby obtaining respective chemical property parameter Pc as a form of the property parameter P.

The computing device 2 and the laboratory system 5 can be connected to each other via a transmission line 9 for transmitting energy and/or information, such as computational data, or alike, between the computing device 2 and the laboratory system 5. When carrying out the assessment program 3, the computing device 2 provides an assessment and development module 10 for managing, handling, processing and/or visualising and thus operating all data used in connection with the assessment method and/or training method as described herein. The assessment and development module 10 can be provided with data from the laboratory system 5 and can provide data thereto for developing chemical substances X. Such data may comprise optimisation parameters O for optimising property parameters P by altering the chemical substances X according to respective optimisation needs.

The assessment and development module 10 may comprise a computational chemistry engine 11, a database 12, a prediction engine 13, an impact approximation unit 14, a performance approximation unit 15, and a design generator 16. In operation, the computational chemistry engine 11 can provide or suggest a chemical representation C, such as a chemical nomenclature, of the chemical substance X, and can analyse the chemical substance X and provide a molecular structure M and latent representation D thereof. The database 12 may provide data regarding a reference substance S having a reference structure Q and reference parameters R. The data may be provided along with respective chemical representations C and latent representations L.

The prediction engine 13 performs the prediction of the property parameter P by deriving the property partner P from a comparison between the reference substance A with reference parameter R the and the chemical substance X. While the impact approximation unit 14 can approximate an impact of the chemical X on any other substance based on the property parameter P, such as potential impacts on the environment, organisms, technical systems, etc., The performance approximation unit 15 can approximate a certain performance of the chemical substance X, such as its half-life period, decomposition behaviour, temperature resistance, pressure resistance, chemical energy, weight, density, or alike. Based on respective impact data and performance data obtained from the impact approximation unit 14, and the performance approximation unit 15, the design generator 16 can suppose the optimisation parameter O, for example by suggesting a certain latent representation of an alternative and/or altered chemical substance X' which can be fed back to the computational chemistry engine 11 for further analysis, alteration, recombination, etc.

Fig. 2 shows a schematic illustration of a molecular structure M of the chemical substance X in chemical representation C. The molecular structure M comprises respective atoms A and bonds B therebetween. In the present example, the chemical representation C of Bromotrifluoromethane consisting of a carbon atom in the centre and three fluor and one bromine atom connected to the carbon atom via respective covalent bonds B is illustrated.

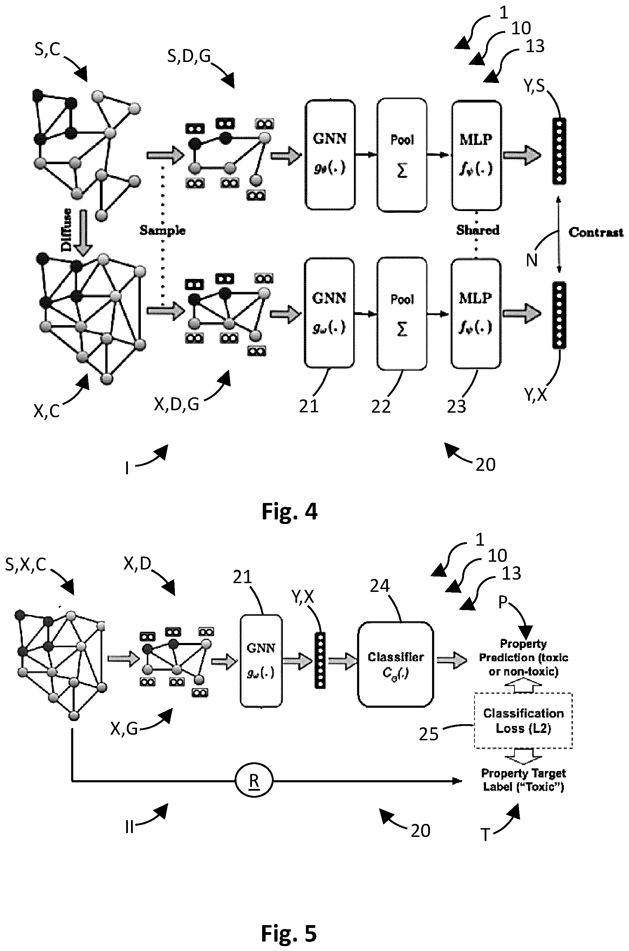

Fig. 3 shows a schematic illustration of the molecular structure M of the chemical substance X shown in Fig. 2 as a latent representation L. Here, the atoms A are represented by nodes and the bonds B therebetween are represented by graphs forming a graphic representation G based on the latent representation L providing respective values embedded in the graphic representation G. Such representation can be used for graph neural networks (GNN) which may be implemented in the assessment and development module 10 and all components thereof (see Fig. 4).

Fig. 4 shows a schematic diagram illustrating supervised learning step the first training phase I which may be implemented as an artificial intelligence (AI) algorithm 20 of the assessment and development module 10, in particular the prediction engine 13 thereof. The AI algorithm 20 can comprise a GNN 21, pooling node 22, a multilayer perceptron (MLP) 23. Chemical representations C of the reference substance S and the chemical substance X can be transformed into their respective latent representations D and graphic representations G which are then each fed to the GNN 21. The output of the GNN 21 is provided to the pooling node 22. The output of the pooling node 22 is provided to the MLP 23. The MLP 23 provides an output value Y for the reference substance S and the chemical substance X which may be compared to each other to identify the contrast therebetween enabling to assess similarity through a similarity score N.

Fig. 5 shows a schematic diagram illustrating a second learning phase II downstream of the first learning phase I. Here, the chemical representation C of the chemical substance X which at the same time can be a reference substance S is transformed into the respective the output value Y is fed into its respective latent representation D and thus the graphic representation G. The graphic representation is fed into the GNN 1 which provides another output value Y. The output value Y is provided to a classifier 24 of the AI algorithm 20. The classifier 24 predicts at least one property parameter P of the chemical substance X.

A discriminator 25 which can also be implemented as a part of the AI algorithm 20 can use respective reference parameter R of the reference substance S previously known. Based on the reference parameter R, the discriminator 25 can derive a target parameter T to be compared to the at least one property parameter P by the discriminator 25. Based on any deviation of the at least one property parameter P and the target parameter T identified by the discriminator 25, the discriminator 25 can issue a loss L, in particular a classification loss which can be fed back to the AI algorithm 20 in order to train and thereby enhance the quality of prediction of the at least one property parameter P.

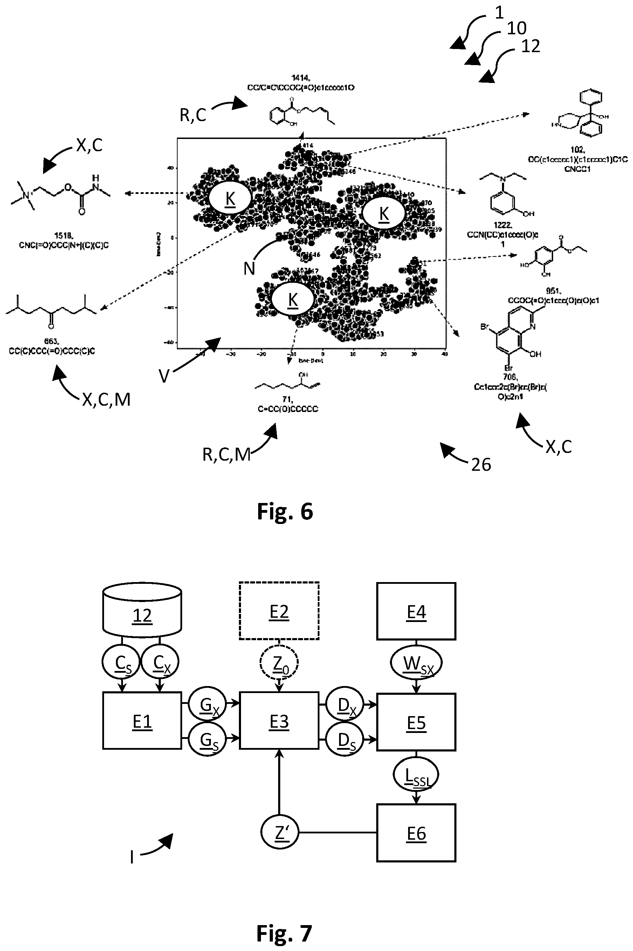

Fig. 6 shows a schematic illustration of a number of molecular structures M of chemical substances X and/or reference substances R arranged in a virtual three-dimensional space V according to their respective similarities which can be expressed by the similarity values N by a graphic engine 26 of the assessment and development module 10. The data for generating the virtual three-dimensional space V containing the similarity values N for the molecular structures X can be organised in the database 12. There, certain groups of molecular structures X may be grouped as clusters K according to certain similar property parameters P or other similarities, such as chemical classifications, that chemical substances X and/or reference substances S assigned to each cluster share.

Fig. 7 shows a schematic block diagram illustrating steps E of a first training phase I where desired and/or required capabilities of the prediction engine 13 are trained by helping the AI engine to emerge. In a first step E1, chemical representations C, for example in the form of the standard nomenclature of the Simplified Molecular Input Line Entry System (SMILES, see https://archive.epa.gov/med/med_archive_03/web/html/smiles.html) taken from the database 12 are converted to respective graphic representations G. In a step E2, a trainable parameter Z can be initialised, for example by providing the trainable parameter Z as a random value for starting value of the trainable starting parameter $Z_0$. In a step E3, the latent representations D of the reference substances S and/or chemical substances X and can be provided using the graphic representations G thereof and the trainable parameter Z for encoding as follows:

$$D_S = ENCODE_Z (G_S)$$

$$D_X = ENCODE_Z (G_X)$$

In a step E4, a pairwise similarity score W can be calculated, for example as a Tanimoto score, for the respective reference substance S and chemical substance X, e.g., in a value range of 0 to 1, as the specific similarity score $W_{XS}$. The specific similarity score $W_{SX}$ is then issued for the respective pair of reference substance S and chemical substance X. For calculating the similarity score W, respective chemical features determining the similarity scores W of the respective reference substance S and chemical substance X can be managed and stored in the database 12.

In a step E5, the respective loss L is calculated for all pairs of reference substances S and chemical substances X in the database 12 based on the respective latent representations $D_S$, $D_X$. The loss L is high, if a distance $dist(D_S,D_X)$ between the latent representations D is high, while the similarity score $W_{SX}$ between the reference substance S and the chemical substance X is low, as well as if the distance between the latent representations D is low, while the similarity score between the reference substance as in the chemical substance X is high. The loss L can be calculated through SSL as a sub supervised learning loss $L_{SSL}$ as follows:

$$L_{SSL} = \left[ \log \frac{\sum \exp(dist(E_X, E_Y) \cdot similarity\_score(X,Y))}{\sum \exp(dist(E_X, E_Y) \cdot (1 - similarity\_score(X,Y)))} \right]$$

In a step E6, the trainable parameter Z can be optimised by finding an optimised trainable parameter Z' for all pairs of reference substances S and chemical substances X in the database 12, such that a total loss $L_{total}(L_{SSL})$ for all available pairs of reference substances S and chemical substances X reaches a current minimum. The optimised trainable parameter Z' can then be fed back to step E3 for performing a respective latent encoding. The optimised trainable parameter Z' can then be used again as the trainable parameter Z for starting another optimisation cycle (Z = Z'). Thereby, training phase I enables to train the prediction engine 13, in particular the AI algorithm 20 thereof, so that it learns to organise molecular structures M that are similar to each other on respective clusters K. The encoding can thus be pushed far for being appliable to chemical substances X having different and possibly previously unknown molecular structures M.

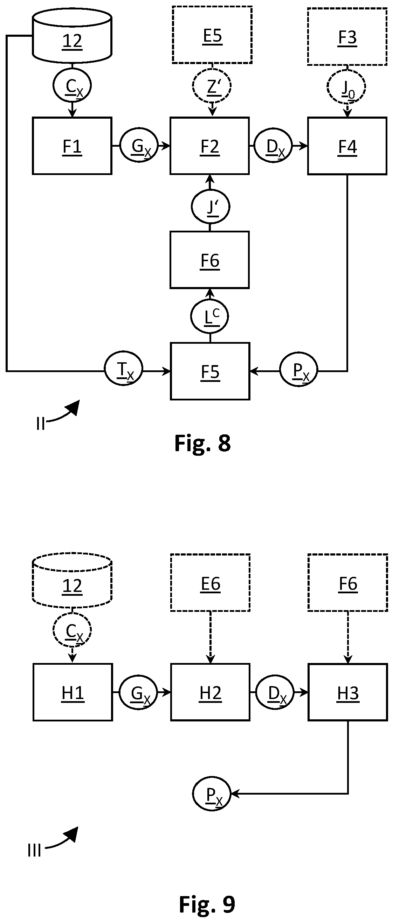

Fig. 8 shows a schematic block diagram illustrating steps F of the second training phase III. In a step F1, a chemical representation $C_X$ of the chemical substance X, for example in the form of the standard nomenclature of SMILES taken from the database 12, is converted to respective graphic representations G. In a step F2, the latent representations D of the or chemical substance X and can be provided using the graphic representations G thereof and the optimized trainable parameter Z' obtained from Step E6 of training phase I for encoding as follows:

$$D_X = ENCODE_{Z'} (G_X)$$

In a step F3, another trainable parameter J can be initialised, for example by providing the trainable parameter J as a random value for starting value of the other trainable starting parameter $J_0$. In a step F4, the property parameter $P_X$ of at least one of the chemical substances X is predicted using the respective latent representation $D_X$ thereof with the help of the prediction engine 13 as follows:

$$P_X = Property\text{-}Predictor_J(D_X)$$

In a step F5, the respective loss L is calculated for the respective predicted property parameter $P_X$ and a corresponding target parameter $T_X$ obtained from the database 12 for the chemical substance X. The loss L is high, if the predicted property parameter $P_X$ and the corresponding target parameter $T_X$ are different. The losses low, if the predicted property parameter $P_X$ and the corresponding target parameter $T_X$ are close to each other, i.e., the same or at least essentially the same. Here, the loss L can be calculated as a cross entropy loss $L^C$ for all property parameters P and target parameters T available in the database 12 as follows:

$$L^C = cross\_enropy\_loss (T_X,P_X)$$

In a step F6, the trainable parameter J can be optimised by finding an optimised trainable parameter J' for all chemical substances X and property parameters P with respective target parameters T in the database 12, such that a total loss $L_{total}(L^C)$ is close to a current minimum. The optimised trainable parameter J' can then be fed back to step F2 for performing a respective latent encoding. The optimised trainable parameter J' can then be used again as the trainable parameter J for starting another optimisation cycle (J = J'). Thereby, training phase II enables to train the prediction engine 13, in particular the AI algorithm 20 thereof, so that it learns to understand property parameters P of molecular structures M based on their respective latent encoding D.

Fig. 9 shows a schematic block diagram illustrating steps H of an application phase III making use of the AI algorithm 20 as it has been trained in training phase I and training phase II. In a step H1, a chemical representation $C_X$ of a chemical substance X, for example, a previously unknown chemical substance X, may be obtained in the form of the standard nomenclature of SMILES from the database 12, and is converted to respective graphic representations G. In a step H2, the latent representations D of the or chemical substance X and can be provided using the graphic representations G thereof and the trainable parameter Z obtained from Step E6 of training phase I for encoding by means of the trained AI engine 13 as follows:

$$D_X = \text{ENCODE}_Z(G_X)$$

In a step H3, the latent representations D of the or chemical substance X can be provided the prediction engine 13, including the now trained AI algorithm 20, along with optimised trainable parameter J from step F6. The prediction engine 13 can then predict the property parameter $P_X$ of the chemical substance X using the respective latent representation $D_X$ with the help of the trained AI algorithm 20 and using respective encoding based on the trainable parameter J as follows:

$$P_X = \text{Property-Predictor}_J(D_X)$$

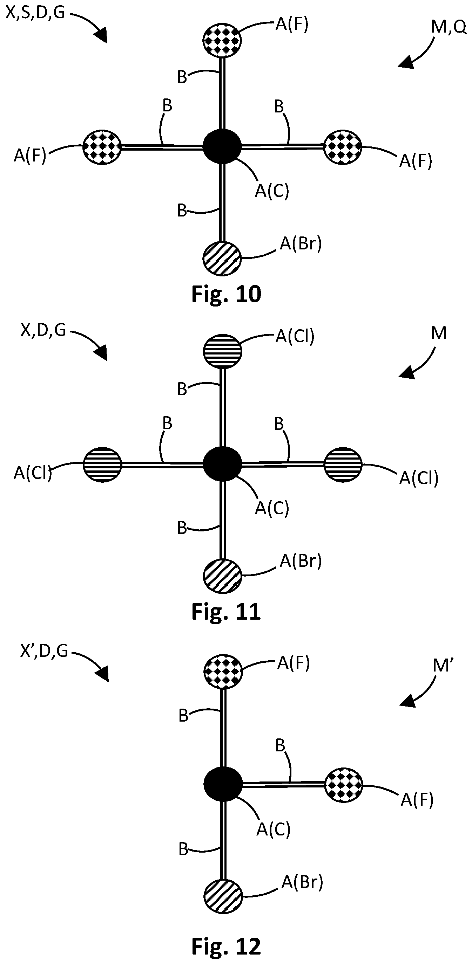

Fig. 10 shows a schematic illustration of the chemical substance X, namely Bromotrifluoromethane ($CBrF_3$) shown in Fig. 2 as another latent representation D. Through sufficient assessment by means of the assessment and development module 10 through the use of the prediction engine 13, the impact approximation unit 14, and/or the performance approximation unit 15, the chemical substance X has been defined as a reference substance R. As such, the reference substance R can be provided in the database 12 with the respective chemical representation C, for example, as SMILES, in the following form: BrC(F)(F)F.

Fig. 11 shows a schematic illustration of a different chemical substance X as a latent representation D. In the present example, the chemical substance X may be Bromotrichloromethane ($CBrCl_3$). The at least one property parameter P of the chemical substance X may derived from the respective reference parameter R of the reference substance S by comparing their molecular structure M and reference structure Q, respectively, in the application phase III. the chemical substance X can be provided in the database 12 with the respective chemical representation C, for example, as SMILES, in the following form: ClC(Cl)(Cl)Br.

Fig. 12 shows a schematic illustration of a different chemical substance X' as a latent representation which has been derived from the chemical substance X shown in Fig. 10 by augmentation. The respective documented graphic representation G can be generated by randomly removing/adding atoms A and bonds B, i.e., nodes and graphs, respectively, from the chemical substance X. This, however, may lead to augmented molecule structures M' which are unrealistic, i.e., in that the number B of bonds does not match the valency of the atoms A involved. Thus, it may be advantageous, to operate the assessment and development module 10 without augmentation.

List of Reference Signs

1　　assessment arrangement
2　　computing device
3　　assessment program
4　　computer-readable data carrier
4a　　computer-readable medium
4b　　data carrier signal
5　　laboratory system
6　　reactor device
7　　physical analysis device
8　　chemical analysis device
9　　transmission line

| | |
|---|---|
| 10 | assessment and development module |
| 11 | computational chemistry engine |
| 12 | database |
| 13 | prediction engine |
| 14 | impact approximation unit |
| 15 | performance approximation unit |
| 16 | design generator |

| | |
|---|---|
| 20 | artificial intelligence algorithm |
| 21 | graph neural network |
| 22 | pooling node |
| 23 | multilayer perceptron |
| 24 | classifying unit |
| 25 | discriminator |
| 26 | graphic engine |

| | |
|---|---|
| I | first training phase |
| II | second training phase |
| III | application phase |

| | |
|---|---|
| A | Atom |
| B | Bond |
| C | chemical representation / nomenclature |
| D | latent representation |
| G | graphic representation |
| J | trainable parameter |
| $J_0$ | starting parameter |
| K | cluster |
| L | loss |
| M | molecular structure |
| N | similarity value |
| O | optimisation parameter |
| P | property parameter |
| $P_P$ | physical property |
| Pc | chemical property |
| Q | reference structure |
| R | reference parameter |
| S | reference substance |
| T | target parameter |
| V | virtual space |
| W | similarity score |
| X | chemical substance |
| Y | output value |
| Z | trainable parameter |
| $Z_0$ | starting parameter |

| | |
|---|---|
| E1 | create graphic representations |
| E2 | initialise trainable parameter |
| E3 | calculate latent encoding |
| E4 | calculate similarity score |
| E5 | calculate loss (SSL) |
| E6 | optimise trainable parameter |

| | |
|---|---|
| F1 | create graphic representation |
| F2 | calculate latent encoding |
| F3 | initialise trainable parameter |
| F4 | predict property parameter |
| F5 | calculate loss (cross entropy) |

F6    optimise trainable parameter

H1    create graphic representation
H2    calculate latent encoding
H3    predict property parameter

**Claims**

1.  Assessment method for computer assisted assessment of at least one property parameter (P) of a chemical substance (X), comprising the steps of

    obtaining a molecular structure (M) of the chemical substance (X); obtaining at least one reference structure (Q) of at least one reference substance (S) having at least one reference parameter (R),
    comparing the molecular structure (M) to the at least one reference structure (Q); and
    deriving the at least one property parameter (P) from the comparison based on the at least one reference parameter (R).

2.  Assessment method according to claim 1, wherein the step of comparing involves identifying a similarity between the molecular structure (M) and the at least one reference structure (Q).

3.  Assessment method according to claim 1 or 2, wherein the step of comparing involves calculating a similarity score (W) of the molecular structure (M) and the at least one reference structure (Q).

4.  Assessment method according to at least one of claims 1 to 3, wherein the steps of obtaining the molecular structure (M) and/or of the at least one reference structure (Q) involves providing a latent representation (D) of the chemical substance (X) and/or of the at least one reference substance (S), respectively.

5.  Assessment method according to claim 4, wherein the step of comparing involves comparing the latent representation (D) of the molecular structure (M) to the latent representation (D) of the at least one reference structure (Q).

6.  Assessment method according to at least one of claims 1 to 5, further comprising the step of selecting the at least one reference structure (Q) of the at least one reference substance (S) from a reference database (12).

7.  Assessment method according to at least one of claims 1 to 6, wherein at least one the reference structure (Q) and/or the at least one reference substance (S) is selected from a reference cluster (K) containing reference structures (Q) and/or reference substances (S), respectively, being relatively similar to each other.

8.  Assessment method according to at least one of claims 1 to 7, wherein the step of comparing and/or the step of deriving involves predicting the at least one property parameter (P) by means of a trained prediction algorithm (20) comprising at least one trainable parameter (J, Z).

9.  Assessment method according to claim 8, further comprising the step of training the prediction algorithm (20) by adjusting the trainable parameter (J, Z) through an assessment evaluation of a quality of the prediction of a similarity parameter (N) and/or the at least one property parameter (P) based on a comparison of the at least one reference substance (S) with another reference substance (S) having at least one predetermined similarity score (W) and/or reference parameter (R), respectively, that serves as a target parameter (T) for the predicted similarity parameter and/or property parameter (P), respectively.

10. Assessment method according to claim 8 or 9, wherein in a first training phase (I), the step of comparing the molecular structure (M) to the at least one reference structure (Q) is being trained.

11. Assessment method according to at least one of claims 9 to 10, wherein in a second training phase (II), deriving the at least one property parameter (P) from the comparison based on the at least one reference parameter (R) is being trained.

12. An assessment program (3) comprising instructions which, when the program is executed by a computing device (2), cause the computing device (2) to carry out an assessment method according to at least one of claims 1 to 11.

13. A computer-readable data-carrier (4, 4a, 4b) having stored thereon the assessment program (3) of claim 12.

14. A computing device (2) configured to carry out an assessment program (3) according to claim 12 and/or comprising a computer-readable data-carrier (4, 4a, 4b) according to claim 13.

15. Assessment arrangement (1) for a computer assisted assessment of at least one property parameter (P) of a chemical substance (X), configured to carry out the assessment method according to at least one of claims 1 to 11, comprising an assessment program (3) according to claim 12, comprising a computer-readable data-carrier (4, 4a, 4b) according to claim 13, and/or comprising a computing device (2) according to claim 14.

**Fig. 1**

**Fig. 2**

**Fig. 3**

**Fig. 4**

**Fig. 5**

**Fig. 6**

**Fig. 7**

**Fig. 8**

**Fig. 9**

Fig. 10

Fig. 11

Fig. 12

| Europäisches Patentamt<br>European Patent Office<br>Office européen des brevets | **PARTIAL EUROPEAN SEARCH REPORT**<br>under Rule 62a and/or 63 of the European Patent Convention.<br>This report shall be considered, for the purposes of<br>subsequent proceedings, as the European search report | **Application Number**<br>EP 23 22 0577 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HUI LIU ET AL: "Attention-wise masked graph contrastive learning for predicting molecular property", ARXIV.ORG, CORNELL UNIVERSITY LIBRARY, 201 OLIN LIBRARY CORNELL UNIVERSITY ITHACA, NY 14853, 2 May 2022 (2022-05-02), XP091247313, * the whole document * | 1-15 | INV.<br>G16C20/30<br><br>ADD.<br>G16C20/70 |
| X | TEDDY KOKER ET AL: "Graph Contrastive Learning for Materials", ARXIV.ORG, CORNELL UNIVERSITY LIBRARY, 201 OLIN LIBRARY CORNELL UNIVERSITY ITHACA, NY 14853, 24 November 2022 (2022-11-24), XP091377558, * the whole document *<br><br>-/-- | 1-15 | |

**TECHNICAL FIELDS SEARCHED (IPC)**

G16C
G06N

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 4 October 2024 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

page 1 of 2

Europäisches Patentamt

European Patent Office

Office européen des brevets

**PARTIAL EUROPEAN SEARCH REPORT**

Application Number

EP 23 22 0577

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | YAOCHEN XIE ET AL: "Self-Supervised Learning of Graph Neural Networks: A Unified Review", ARXIV.ORG, CORNELL UNIVERSITY LIBRARY, 201 OLIN LIBRARY CORNELL UNIVERSITY ITHACA, NY 14853, 25 April 2022 (2022-04-25), XP091189860, * the whole document * | 1-15 | |
| X | PENGCHENG JIANG ET AL: "Bi-level Contrastive Learning for Knowledge-Enhanced Molecule Representations", ARXIV.ORG, CORNELL UNIVERSITY LIBRARY, 201 OLIN LIBRARY CORNELL UNIVERSITY ITHACA, NY 14853, 29 September 2023 (2023-09-29), XP091627066, * the whole document * | 1-15 | TECHNICAL FIELDS SEARCHED (IPC) |
| X | KISUNG MOON ET AL: "3D Graph Contrastive Learning for Molecular Property Prediction", ARXIV.ORG, CORNELL UNIVERSITY LIBRARY, 201 OLIN LIBRARY CORNELL UNIVERSITY ITHACA, NY 14853, 31 May 2022 (2022-05-31), XP091292872, * the whole document * | 1-15 | |

EPO FORM 1503 03.82 (P04C10)

Europäisches
Patentamt

European
Patent Office

Office européen
des brevets

**INCOMPLETE SEARCH
SHEET C**

Claim(s) completely searchable:
-

Claim(s) searched incompletely:
    1-15

Reason for the limitation of the search:

In his letter received 06-09-2024, the applicant considered that a search in respect of the entire scope of claims 1 to 15, as originally filed could be carried out. It is considered that the applicant failed to provide arguments as to why the claims were supported by the description and could be carried out on their whole breath by the skilled person as argued. In particular, step E4 is a step of the application defined in the description, therefore the step is defined. It has further not been argued that the whole application could not be carried out by a skilled person but only that a skilled person would not be able to carry out a property determination for a molecule on substantially the whole scope of the claim as worded without undue burden.

Consequently, the subject-matter of the claims is still considered not supported by the description (Article 84 EPC) to such an extent and not sufficiently disclosed to be carried out by skilled person on the whole breath of the claims (Article 83 EPC) that a meaningful search based on the scope of the claim as worded is not considered as being possible (Rule 63 EPC).

Claim 1 encompasses at least the direct determination of a property of molecule using in an unspecified manner a comparison of the structure of the molecule with the structure of an another molecule to directly derive the property using in an unspecified manner a parameter of the other molecule.

It is considered that there is no support in the disclosed embodiments for such a direct determination. The embodiments use a set of machine learning models. What could be related to the above comparison appears only in step E4 (figure 7; description page 17, lines 6-13) which is a model training step, i.e. a step for training a model generating a molecule latent representation from a graph representing a molecule and not a step providing a measure used in the determination a property of a molecule. Step E4 does not allow to a direct determination of a property of a molecule. In addition, in the description page 21, lines 4-7, one reads "The at least one property parameter P of the chemical substance X may derived from the respective reference parameter R of the reference substance S by comparing their molecular structure M and reference structure Q, respectively, in the application phase III". The "application phase III" is disclosed with reference to figure 9. This phase takes only as input the chemical substance X which is processed by the machine learning model trained in the previous phases (figure 7 and 8) without any comparison with a reference substance S. The disclosed embodiments does not appear to fall into the scope of claim 1.

Consequently, even if one could formulate an interpretation of claim 1 in which the disclosed embodiments could fit (such an interpretation could not be achieved and does not appear possible), the scope of claim 1 is considered to encompass interpretations that have no support in the description to such an extent (e.g. as argued in the paragraph above) that carrying out a search based scope of claim 1 as worded does not

Europäisches
Patentamt

European
Patent Office

Office européen
des brevets

**INCOMPLETE SEARCH
SHEET C**

Application Number

EP 23 22 0577

appear meaningful.

Furthermore, the scope of claim 1 encompasses the prediction of any possible property of a molecule by direct comparison (e.g. using a distance or similarity measure) of the structure of the molecule and the structure of another molecule using the known property (parameter) of the other molecule. The application does not disclose any manner of carrying out this prediction, i.e. how to determine a similarity measure that would allow knowing the property of molecule to determine the property of another molecule using only the result of the similarity measure and the known property.

Consequently, a skilled person would not be able to carry out a property determination for a molecule on substantially the whole scope of the claim without undue burden (Article 83 EPC).

The scope of the dependent claims adds only partial elements of the disclosed property prediction machine learning framework that do not alleviate any of the above mentioned issues.

Consequently, it is considered that a meaningful search cannot be carried for the claimed subject-matter as worded.

Consequently, considering the wording of the above mentioned passage in the description on page 21, lines 4-7 in relation to the wording of claim 1, which would appear to indicate that the used wording would refer to the property prediction architecture disclosed with respect to figures 7, 8 and 9, the scope of claims will be understood as the molecule property prediction machine learning architecture of figures 7, 8 and 9 and corresponding section of the description. The scope of the search has been restricted accordingly.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WANG, Y ; WANG, J ; CAO, Z. et al.** Molecular contrastive learning of representations via graph neural networks. *Nat Mach Intell*, 2022, vol. 4, 279-287

- **WANG Y ; MAGAR R ; LIANG C ; BARATI FARIMANI A**. Improving Molecular Contrastive Learning via Faulty Negative Mitigation and Decomposed Fragment Contrast. *J Chem Inf Model.*, 31 May 2022, vol. 62 (11), 2713-2725